(19) Europäisches Patentamt / European Patent Office / Office européen des brevets

(11) **EP 4 332 079 A1**

(12) **EUROPEAN PATENT APPLICATION**
published in accordance with Art. 153(4) EPC

(43) Date of publication:
**06.03.2024 Bulletin 2024/10**

(21) Application number: **22795842.8**

(22) Date of filing: **27.04.2022**

(51) International Patent Classification (IPC):
**C07C 17/363** $^{(2006.01)}$   **A62D 3/40** $^{(2007.01)}$
**C07C 19/08** $^{(2006.01)}$

(52) Cooperative Patent Classification (CPC):
**A62D 3/40; C07C 17/363; C07C 19/08**

(86) International application number:
**PCT/JP2022/019051**

(87) International publication number:
**WO 2022/230929 (03.11.2022 Gazette 2022/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.04.2021   JP 2021077490**

(71) Applicant: **AGC INC.**
**Chiyoda-ku,**
**Tokyo 1008405 (JP)**

(72) Inventor: **FUJITA Tomoyuki**
**Tokyo 100-8405 (JP)**

(74) Representative: **Müller-Boré & Partner**
**Patentanwälte PartG mbB**
**Friedenheimer Brücke 21**
**80639 München (DE)**

(54) **METHOD FOR DECOMPOSING POLYFLUOROCARBOXYLIC ACIDS**

(57) The present invention relates to a method for decomposing polyfluorocarboxylic acids, characterized in that a mixture containing polyfluorocarboxylic acids, water, and a basic compound is heated.

EP 4 332 079 A1

**Description**

[Technical Field]

The present invention relates to a method for decomposing polyfluorocarboxylic acids.
Priority is claimed on Japanese Patent Application No. 2021-077490, filed April 30, 2021, the content of which is incorporated herein by reference.

[Background Art]

In recent years, due to concerns about the environmental persistence of fluorine-containing organic compounds that have been used as surfactants and surface treatment agents, there is a movement to reduce fluorocarboxylic acids from various fluororesin products, other industrial products, drinking water, factory wastewater, sewage, and the like.
These fluorine-containing organic compounds are highly stable and difficult to decompose since they are formed from carbon-fluorine bonds with high binding energy. For example, Non-Patent Document 1 describes that when heated at 307°C for 65 hours, they decompose by 4%.
From such a background, various studies have been conducted, particularly on the method for decomposing polyfluorocarboxylic acids. For example, a method of reacting with iron powder at a high temperature of 350°C (Patent Document 1), a method of decomposing by reaction with hydrogen peroxide (Patent Document 2), a method of heating with peroxodisulfate ions (Patent Document 3), and the like have been known.
However, the method described in Patent Document 1 requires a very hightemperature reaction, and there are significant equipment restrictions for industrial implementation. In the method described in Patent Document 2, the decomposition rate of polyfluorocarboxylic acid is low. In the method described in Patent Document 3, an excessive amount of peroxodisulfate, which is an oxidizing substance, is required in order to achieve a high decomposition rate, resulting in costs and management.

[Citation List]

[Patent Documents]

[Patent Document 1] Japanese Unexamined Patent Application, First Publication No. 2006-306736
[Patent Document 2] Japanese Unexamined Patent Application, First Publication No. 2006-169146
[Patent Document 3] Japanese Unexamined Patent Application, First Publication No. 2008-285449

[Non-Patent Document]

[Non-Patent Document 1] Journal of Fluorine Chemistry 126 (2005) 1510-1516

[Summary of Invention]

[Technical Problem]

As a method for decomposing polyfluorocarboxylic acids, since conventional methods have insufficient decomposition capacity, and the cost burden is high, a method that enables easier decomposition is required.
In view of the above circumstances, the present invention has an object of providing a method for efficiently decomposing polyfluorocarboxylic acids in a simpler and cheaper manner.

[Solution to Problem]

As a result of intensive studies, the inventors of the present invention have found that the above problems can be solved by the following configuration. More specifically, the inventors of the present invention have found that the capacity for decomposing polyfluorocarboxylic acids is improved when a mixture containing polyfluorocarboxylic acids, water, and a basic compound is heated.

[1] A method for decomposing a polyfluorocarboxylic acid, the method characterized by heating a mixture containing the polyfluorocarboxylic acid, water, and a basic compound, wherein a mass ratio of the polyfluorocarboxylic acid and water (polyfluorocarboxylic acid: water) of 1: $1 \times 10^{15}$ to 1: 0.01 is preferred, 1: $1 \times 10^{12}$ to 1: 0.1 is more preferred, 1: $1 \times 10^9$ to 1: 1 is still more preferred, and 1: $1 \times 10^6$ to 1: 10 is particularly preferred.

[2] The decomposition method according to [1], wherein the basic compound is at least one basic compound selected from the group consisting of sodium hydroxide, potassium hydroxide, calcium hydroxide, sodium carbonate, potassium carbonate, calcium carbonate, sodium hydrogen carbonate, and potassium hydrogen carbonate, and sodium hydroxide and potassium hydroxide are preferred, and potassium hydroxide is more preferred.

[3] The decomposition method according to [1] or [2], wherein a pH of the mixture is 10 or more, preferably 10 to 14, more preferably 12 or more, and still more preferably 12 to 14.

[4] The decomposition method according to any one of [1] to [3], wherein a total amount of metal powders, hydrogen peroxide, ozone, and peroxodisulfate ions is 1% by mass or less with respect to a total mass of the mixture, and the mixture preferably does not contain a metal powder, hydrogen peroxide, ozone, and a peroxodisulfate ion.

[5] The decomposition method according to any one of [1] to [4], wherein a temperature in the heating is higher than 150°C and lower than 195°C, preferably from 155 to 190°C, more preferably from 160 to 185°C, and still more preferably from 165 to 180°C, and a heating time is preferably from 1 to 24 hours, more preferably 1 hour or more and less than 12 hours, and still more preferably 1 hour or more and less than 6 hours.

[6] The decomposition method according to any one of [1] to [5], wherein the polyfluorocarboxylic acid has a fluorinated alkyl group of 4 to 16 carbon atoms, preferably has a fluorinated alkyl group of 5 to 15 carbon atoms, and more preferably has a fluorinated alkyl group of 6 to 14 carbon atoms.

[7] The decomposition method according to any one of [1] to [6], wherein the polyfluorocarboxylic acid is a perfluorocarboxylic acid or a salt thereof, and preferably nonafluoropentanoic acid, perfluorooctanoic acid, perfluorononanoic acid, perfluorodecanoic acid, perfluoroundecanoic acid, perfluorododecanoic acid or a salt thereof.

[8] The decomposition method according to [7], wherein the perfluorocarboxylic acid is perfluorooctanoic acid.

[9] The decomposition method according to any one of [1] to [8], wherein a decomposition product of the polyfluorocarboxylic acid is a fluorinated compound represented by the following formula (D1).

$$\text{Rf-H} \qquad \text{(D1)}$$

In the formula (D1), Rf is a fluorinated alkyl group of 3 to 15 carbon atoms which may have an ethereal oxygen atom.

A method for producing a fluorinated compound, the production method characterized by heating a mixture containing a polyfluorocarboxylic acid, water, and a basic compound, and a mass ratio of the polyfluorocarboxylic acid and water (polyfluorocarboxylic acid: water) is preferably from 1: $1 \times 10^{15}$ to 1: 0.01, more preferably from 1: $1 \times 10^{12}$ to 1: 0.1, still more preferably from 1: $1 \times 10^9$ to 1: 1, and particularly preferably from 1: $1 \times 10^6$ to 1: 10.

The production method according to [10], wherein the basic compound is at least one basic compound selected from the group consisting of sodium hydroxide, potassium hydroxide, calcium hydroxide, sodium carbonate, potassium carbonate, calcium carbonate, sodium hydrogen carbonate, and potassium hydrogen carbonate, and sodium hydroxide and potassium hydroxide are preferred, and potassium hydroxide is more preferred.

The production method according to [10] or [11], wherein a pH of the mixture is 10 or more, preferably 10 to 14, more preferably 12 or more, and still more preferably 12 to 14.

The production method according to any one of [10] to [12], wherein a total amount of metal powders, hydrogen peroxide, ozone, and peroxodisulfate ions is 1% by mass or less with respect to a total mass of the mixture, and the mixture preferably does not contain a metal powder, hydrogen peroxide, ozone, and a peroxodisulfate ion.

The production method according to any one of [10] to [13], wherein a temperature in the heating is higher than 150°C and lower than 195°C, preferably from 155 to 190°C, more preferably from 160 to 185°C, and still more preferably from 165 to 180°C, and a heating time is preferably from 1 to 24 hours, more preferably 1 hour or more and less than 12 hours, and still more preferably 1 hour or more and less than 6 hours.

The production method according to any one of [10] to [14], wherein the polyfluorocarboxylic acid has a fluorinated alkyl group of 6 to 16 carbon atoms, preferably has a fluorinated alkyl group of 5 to 15 carbon atoms, and more preferably has a fluorinated alkyl group of 6 to 14 carbon atoms.

The production method according to any one of [10] to [15], wherein the polyfluorocarboxylic acid is a perfluorocarboxylic acid or a salt thereof, and preferably nonafluoropentanoic acid, perfluorooctanoic acid, perfluorononanoic acid, perfluorodecanoic acid, perfluoroundecanoic acid, perfluorododecanoic acid or a salt thereof.

The production method according to [16], wherein the perfluorocarboxylic acid is perfluorooctanoic acid.

The production method according to any one of [10] to [17], wherein a decomposition product of the polyfluorocarboxylic acid is a fluorinated compound represented by the following formula (D1).

Rf-H            (D1)

In the formula (D1), Rf is a fluorinated alkyl group of 3 to 15 carbon atoms which may have an ethereal oxygen atom.

[Advantageous Effects of Invention]

According to the method of the present invention, polyfluorocarboxylic acids can be efficiently decomposed in a simpler and cheaper manner.

[Description of Embodiments]

A first aspect of the present invention is a method for decomposing perfluorocarboxylic acids in which a mixture containing polyfluorocarboxylic acids, water, and a basic compound is heated.
The term "polyfluorocarboxylic acids" means a compound (polyfluorocarboxylic acid) in which a monovalent organic group having two or more fluorine atoms is bonded to a carboxy group, and a salt and precursor thereof.
Examples of the above salt include alkali metal salts, alkaline earth metal salts and ammonium salts of polyfluorocarboxylic acid, and lithium salts, sodium salts, potassium salts, ammonium salts, calcium salts, and magnesium salts are preferred, and lithium salts, sodium salts, potassium salts, and ammonium salts are more preferred.
Examples of the above precursor include fluorinated alcohols, fluorinated aldehydes, acid halides having a fluorine atom, and fluorinated carboxylic acid esters. A fluorinated alcohol is a compound in which an oxygen atom of a carbonyl group in a carboxy group of a polyfluorocarboxylic acid is substituted with two hydrogen atoms. A fluorinated aldehyde is a compound in which a hydroxyl group in a carboxy group of a polyfluorocarboxylic acid is substituted with a hydrogen atom. An acid halide having a fluorine atom is a compound in which a hydroxyl group in a carboxy group of a polyfluorocarboxylic acid is substituted with a halogen atom. A fluorinated carboxylic acid ester is a compound in which a hydrogen atom of a hydroxyl group in a carboxy group of a polyfluorocarboxylic acid is substituted with a monovalent hydrocarbon group.
The polyfluorocarboxylic acids of the present embodiment preferably have a fluorinated alkyl group, and more preferably have a perfluoroalkyl group. The fluorinated alkyl group may be a fluorinated alkyl group having an ethereal oxygen atom. A "fluorinated alkyl group having an ethereal oxygen atom" means a fluorinated alkyl group having an oxygen atom inserted between carbon-carbon bonds in the fluorinated alkyl group. The fluorinated alkyl group is preferably bound to a carbon atom of a carbonyl group in the polyfluorocarboxylic acids. The number of carbon atoms in the fluorinated alkyl group or perfluoroalkyl group is preferably from 4 to 16, more preferably from 5 to 15, and still more preferably from 6 to 14. The above polyfluorocarboxylic acid is preferably a carboxylic acid having a perfluoroalkyl group, a salt thereof, or a precursor thereof, and more preferably a carboxylic acid having a perfluoroalkyl group or a salt thereof. It should be noted that the fluorinated alkyl group is a group in which at least two hydrogen atoms of an alkyl group represented by $-C_nH_{2n+1}$ (n is a positive integer) or a cycloalkyl group are substituted with fluorine atoms, and may be linear or branched. A perfluoroalkyl group is a group in which all hydrogen atoms of an alkyl group represented by $-C_nH_{2n+1}$ (n is a positive integer) or cycloalkyl group are substituted with fluorine atoms.
Examples of the polyfluorocarboxylic acid having a fluorinated alkyl group of 4 to 16 carbon atoms include polyfluoropentanoic acid, polyfluorooctanoic acid, polyfluorononanoic acid, polyfluorodecanoic acid, polyfluoroundecanoic acid, polyfluorododecanoic acid, and polyfluorotetradecanoic acid.
Examples of the carboxylic acid having a perfluoroalkyl group include nonafluoropentanoic acid, perfluorooctanoic acid, perfluorononanoic acid, perfluorodecanoic acid, perfluoroundecanoic acid, and perfluorododecanoic acid, and perfluorooctanoic acid is preferred.
The polyfluorocarboxylic acid of the present embodiment is preferably a perfluorocarboxylic acid represented by the following general formula (A1) or a salt thereof.

$$(R^FCOO)_{n1}M \qquad (A1)$$

$R^F$ is a perfluoroalkyl group of 4 to 16 carbon atoms which may have an ethereal oxygen atom. The perfluoroalkyl group is as described above. The perfluoroalkyl group may be linear or branched, or may have a ring structure. The perfluoroalkyl group is preferably linear. Further, the number of carbon atoms described above is preferably from 4 to 16, more preferably from 5 to 15, and still more preferably from 6 to 14. When $R^F$ has an ethereal oxygen atom, the number of ethereal oxygen atoms is preferably from 1 to 4, and more preferably from 1 to 3.
M represents H, Li, Na, K, $NH_4$, Ca, or Mg. When M is H, Li, Na, K, or $NH_4$, n1 is 1, and when M is Ca or Mg, n1 is 2. M is preferably Li, Na, K or $NH_4$. These are water soluble and easy to handle.

In the present embodiment, the polyfluorocarboxylic acids are heated together with water and a basic compound. The polyfluorocarboxylic acids may or may not be dissolved in water, and are preferably dissolved. A mass ratio of polyfluorocarboxylic acids and water (polyfluorocarboxylic acids: water) is preferably from $1: 1 \times 10^{15}$ to $1: 0.01$, more preferably from $1: 1 \times 10^{12}$ to $1: 0.1$, still more preferably from $1: 1 \times 10^{9}$ to $1: 1$, and particularly preferably from $1: 1 \times 10^{6}$ to $1: 10$. In the method of the present embodiment, efficient decomposition is possible even if the concentration of the polyfluorocarboxylic acids is high, and further reduction is possible even if the concentration of the polyfluorocarboxylic acids is low.

In particular, in the regulation on the registration, evaluation, authorisation and restriction of chemicals (REACH), it is required that the concentration of perfluorooctanoic acids is 25 ppb by mass or less. According to the method of the present embodiment, the concentration of perfluorooctanoic acid can be reduced to 25 ppb by mass or less, and even down to about 10 ppb by mass by adjusting the reaction time and reaction temperature.

The amount of the polyfluorocarboxylic acids with respect to the total mass of the mixture is preferably from 0.001 to 5% by mass, more preferably from 0.005 to 4% by mass, and still more preferably from 0.01 to 3% by mass.

The heating temperature is preferably higher than 150°C and lower than 195°C, more preferably from 155 to 190°C, still more preferably from 160 to 185°C, and particularly preferably from 165 to 180°C. When it is higher than 150°C, a sufficient decomposition rate can be ensured, which is suitable for industrial implementation. When it is less than 195°C, the temperature can be easily adjusted by heating steam or the like. The term "heating temperature" means the temperature of the mixture during heating.

The heating time is not particularly limited, and is usually from 1 to 24 hours. According to the method of the present embodiment, polyfluorocarboxylic acids can be sufficiently decomposed in a short period of time of less than 12 hours, or even about less than 6 hours.

Since the polyfluorocarboxylic acids are acidic compounds, especially at high concentrations, a basic compound is added from the viewpoint of further increasing the decomposition rate of the polyfluorocarboxylic acids and preventing corrosion. Examples of such a basic compound include sodium hydroxide, potassium hydroxide, calcium hydroxide, sodium carbonate, potassium carbonate, calcium carbonate, sodium hydrogen carbonate, and potassium hydrogen carbonate, and sodium hydroxide and potassium hydroxide are preferred, and potassium hydroxide is more preferred.

The amount of the basic compound in the mixture is preferably equal to or more than the equivalent of the polyfluorocarboxylic acids. A ratio of the number of moles of the basic compound with respect to the number of moles of the polyfluorocarboxylic acid in the mixture is preferably from 0.01 to 50,000,000, more preferably from 0.1 to 500,000, and still more preferably from 1 to 5,000.

The amount of the basic compound with respect to the total mass of the mixture is preferably 50% by mass or less, more preferably 30% by mass or less, and still more preferably 15% by mass or less. When it is equal to or less than the above upper limit value, it can be easily supplied to a treatment process using a solution of sodium hydroxide, potassium hydroxide, or the like. The amount of the basic compound with respect to the total mass of the mixture is preferably 0.01 % by mass or more, more preferably 0.1% by mass or more, and still more preferably 1% by mass or more. That is, the amount of the basic compound with respect to the total mass of the mixture is preferably from 0.01 to 50% by mass, more preferably from 0.1 to 30% by mass, and still more preferably from 1 to 15% by mass.

When decomposing polyfluorocarboxylic acids by the method of the present embodiment, a material other than the above-described polyfluorocarboxylic acids, water, and basic compound may coexist. Examples of the above material include alcohols, carboxylic acids, and salts of inorganic acids and organic acids. One aspect of the present invention preferably contains methanol. Another aspect of the present invention preferably contains potassium iodide.

When decomposing polyfluorocarboxylic acids by the method of the present embodiment, it is preferable that a metal powder, hydrogen peroxide, ozone, and a peroxodisulfate ion are not substantially contained, and it is more preferable that they are not contained. The expression "not substantially contained" means that the total amount of these is 1% by mass or less with respect to the total mass of the mixture. Examples of the above metal powder include powdered iron, copper, lead, aluminum, and zinc.

A fluorinated compound obtained by decomposing polyfluorocarboxylic acids by the method of the present embodiment is represented by the following formula (D1).

$$\text{Rf-H} \qquad \text{(D1)}$$

In the formula (D1), Rf is a fluorinated alkyl group of 3 to 15 carbon atoms which may have an ethereal oxygen atom. Here, the fluorinated alkyl group is a group as described above. The fluorinated alkyl group may be linear or branched, or may have a ring structure. The fluorinated alkyl group is preferably linear. Further, the number of carbon atoms described above is preferably from 4 to 14, and more preferably from 5 to 13.

The fluorinated compound (D1) is water-insoluble and can be easily separated from the water used for decomposition by layer separation.

The method of the present embodiment may be carried out in the co-presence of nitrogen, air, and other gases, or may be carried out in a state in which these gases are degassed. The operating pressure is not particularly limited, and it may be pressurized or depressurized, but by-products produced by decomposition while reducing the pressure can be removed at the same time. The absolute pressure when reducing the pressure is preferably from 0.001 to 0.099 MPa, more preferably from 0.01 to 0.09 MPa, and still more preferably from 0.1 to 0.09 MPa.

As the material of equipment for carrying out decomposition by the method of the present embodiment, any material that does not corrode the equipment may be employed, and various metals, glasses, and ceramics can be used.

By using the method of the present embodiment, the decomposition rate of polyfluorocarboxylic acids calculated by the following formula based on the mass of the polyfluorocarboxylic acids before decomposition can be set to 90.0% or more, or even can be set to 95.0% or more. The mass of the polyfluorocarboxylic acid in the mixture can be calculated from measurement by a liquid chromatography-mass spectrometry method, which will be described later. As described above, polyfluorocarboxylic acids can be efficiently decomposed by the method of the present embodiment.

$$\text{Decomposition rate (\%)} = ((\text{mass of polyfluorocarboxylic acids before decomposition}) - (\text{mass of polyfluorocarboxylic acids after decomposition})) / (\text{mass of polyfluorocarboxylic acids before decomposition}) \times 100$$

The initial pH of the mixture containing the basic compound before decomposition is preferably 10 or higher, and more preferably 12 or higher. The upper limit of the initial pH is usually 14. That is, the initial pH is preferably from 10 to 14, and more preferably from 10 to 12. When the initial pH is within the above range, the decomposition rate of polyfluorocarboxylic acids is further improved.

A second aspect of the present invention is a method for producing a fluorinated compound by decomposing polyfluorocarboxylic acids to obtain a fluorinated compound.

The details of each compound and the decomposition method thereof, the fluorinated compound and the like are as described above in the first aspect.

By using the method of the present embodiment, the recovery rate of the fluorinated compound, which is the decomposition product of polyfluorocarboxylic acid, calculated by the following formula based on the mass of the polyfluorocarboxylic acids before decomposition can be set to 60.0% or more, or even can be set to 70.0% or more. The mass of the fluorinated compound in the mixture can be determined by a method in which the structure is specified by NMR and then the number of moles is calculated and converted into a mass, a method of calculation in accordance with the liquid chromatography-mass spectrometry method described later, or the like. As described above, according to the method of the present embodiment, a fluorinated compound, which is a decomposition product of polyfluorocarboxylic acids, can be efficiently recovered from a mixture containing the polyfluorocarboxylic acids and water by a simpler and easier method.

$$\text{Recovery rate (\%)} = ((\text{mass of fluorinated compound generated by decomposition})/(\text{molecular weight of fluorinated compound generated by decomposition}))/((\text{mass of polyfluorocarboxylic acids before decomposition})/(\text{molecular weight of polyfluorocarboxylic acids})) \times 100$$

[Examples]

The present invention will be specifically described below with reference to Examples, but the present invention is not limited to these Examples.

[Decomposition rate of polyfluorocarboxylic acid]

The concentration of polyfluorocarboxylic acid in the mixture obtained in each of the following examples was measured by a liquid chromatography-mass spectrometry method. Specific measurement conditions are as follows. It should be noted that a standard solution with a known concentration was prepared, and the concentration of polyfluorocarboxylic acid was calculated by the standard addition method. Using the concentration of polyfluorocarboxylic acid obtained from a mixed solution before and after decomposition, the decomposition rate of polyfluorocarboxylic acid was calculated by the following formula. It should be noted that in mass spectrometry (MS), $C_7F_{15}COO^-$ (molecular ion mass: 413) and $C_7F_{15}^-$ (molecular ion mass: 369) were detected and monitored as a primary ion and a secondary ion, respectively.

The decomposition rate and recovery rate were calculated using the above formulas.

<LC-MS/MS measurement conditions>

Measuring device: Agilent 1260 series HPLC/6460MS

(HPLC conditions)

Column: Cadenza CD-C18 manufactured by Imtakt Corporation, inner diameter: 2 mmΦ, length: 100 mm
Eluent:

(Solution A) 0.002 mol/L aqueous ammonium acetate solution, (Solution B) methanol for HPLC
Flow rate: 0.3 mL/min
Injection volume: 5 μL
Column oven temperature: 35°C
Gradient conditions: time (minutes) from the start of measurement and composition of eluent (solution A/solution B (volume %))
0 minutes (38/62) → 2 minutes (38/62) → 5 minutes (0/100) → 10 minutes (0/100) → 10.01 minutes (38/62) → 20 minutes (38/62)

(MS conditions)

Ionization method: ESI
Nebulizer: Nz
Dry gas: $N_2$ (10 L/min)
Sheath gas: $N_2$ (10 L/min)
Dry gas temperature: 300°C
Sheath gas temperature: 350°C
Detected ion: Negative
Fragmentor voltage: 90V
Collision gas: $N_2$
Collision: 4eV
Monitoring: MRM (m/z = 413 and 369)

[Comparative Example 1]

A mixture was obtained by mixing 0.15 g of perfluorooctanoic acid (PFOA) and 1,500.0 g of distilled water, and was used as a test liquid 1. When the concentration of PFOA in the test liquid 1 was measured, it was 87 ppm by mass.
A 1 L Hastelloy autoclave equipped with a stirrer was depressurized with a vacuum pump, the test liquid 1 was charged in such a manner that air did not enter, the internal temperature was adjusted to 170°C with an oil bath, and the test liquid 1 was held for 5 hours under stirring. After cooling, the concentration of PFOA in the contents was measured and found to be 5.9 ppm by mass, and the PFOA decomposition rate calculated based on the concentration of the charged PFOA was 93.2%.

[Example 1]

1.5 g of PFOA, 1,000.2 g of distilled water, and 2.1 g of a 48% aqueous potassium hydroxide solution were mixed to obtain 1,500 ppm by mass of a PFOA solution (hereinafter referred to as a PFOA solution 1). Furthermore, a mixture was obtained by mixing 54.95 g of the PFOA solution 1, 464.8 g of distilled water, and 31.35 g of a 48% aqueous potassium hydroxide solution, and was used as a test liquid 2. When the concentration of PFOA in the test liquid 2 was measured, it was 154 ppm by mass.

After holding the test liquid 2 at 170°C for 5 hours and then cooling in the same manner as in Comparative Example 1, the concentration of PFOA in the contents was measured and found to be 5.9 ppm by mass, and the PFOA decomposition rate calculated based on the concentration of the charged PFOA was 96.2%.

[Example 2]

A mixture was obtained by mixing 55.21 g of the PFOA solution 1, 434.9 g of distilled water, 30.51 g of a 48% aqueous potassium hydroxide solution, and 30.4 g of potassium iodide, and was used as a test liquid 3. When the concentration of PFOA in the test liquid 3 was measured, it was 170 ppm by mass.

After holding the test liquid 3 at 170°C for 5 hours and then cooling in the same manner as in Comparative Example 1, the concentration of PFOA in the contents was measured and found to be 4.2 ppm by mass, and the PFOA decomposition rate calculated based on the concentration of the charged PFOA was 97.5%.

[Example 3]

A mixture was obtained by mixing 55.04 g of the PFOA solution 1, 215.2 g of distilled water, 32.56 g of the 48% aqueous potassium hydroxide solution, 30.61 g of potassium iodide, and 220.08 g of methanol, and was used as a test liquid 4. When the concentration of PFOA in the test liquid 4 was measured, it was 154 ppm by mass.

After holding the test liquid 4 at 170°C for 5 hours and then cooling in the same manner as in Comparative Example 1, the concentration of PFOA in the contents was measured and found to be 0.3 ppm by mass, and the PFOA decomposition rate calculated based on the concentration of the charged PFOA was 99.8%.

[Example 4]

15.4 g of PFOA, 449.0 g of distilled water, and 50.7 g of a 48% aqueous potassium hydroxide solution were mixed to obtain a test liquid 5, which was a slurry mixture. The above procedure was carried out in the same manner as in Comparative Example 1 with the exception that the test solution 5 was charged by opening the autoclave. The obtained liquid was transparent and separated into two layers, and 10.1 g of the lower layer and 494.75 g of the upper layer were recovered. When the concentration of PFOA in the upper layer was measured, it was 600 ppm by mass. Further, when the decomposition rate of PFOA was calculated, it was 98.1%. It should be noted that when the lower layer was recovered and analyzed by NMR, it was confirmed to be $C_7F_{15}H$, and the recovery rate based on the mass of the charged PFOA was 73.4%.

As shown in Examples 1 to 4, by using the method of the present invention, polyfluorocarboxylic acids can be decomposed at a high decomposition rate, and fluorinated compounds, which are decomposition products of polyfluorocarboxylic acids, can be efficiently recovered.

[Industrial Applicability]

By using the method of the present invention, water-soluble fluorine-containing organic compounds used for surfactants, surface treatment agents, and the like can be easily decomposed, and fluorinated compounds having polyfluoroalkyl groups can be efficiently recovered. Therefore, the method of the present invention is effective in preventing environmental pollution caused by fluorine-containing organic compounds.

**Claims**

1. A method for decomposing a polyfluorocarboxylic acid, comprising heating a mixture containing the polyfluorocarboxylic acid, water, and a basic compound.

2. The method according to Claim 1,
   wherein said basic compound is at least one basic compound selected from the group consisting of sodium hydroxide, potassium hydroxide, calcium hydroxide, sodium carbonate, potassium carbonate, calcium carbonate, sodium hydrogen carbonate, and potassium hydrogen carbonate.

3. The method according to Claim 1 or 2, wherein said mixture has a pH of 10 or higher.

**4.** The method according to any one of Claims 1 to 3, wherein the mixture does not contain a metal powder, hydrogen peroxide, ozone, and peroxodisulfate ions.

**5.** The method according to any one of Claims 1 to 4, wherein a temperature in said heating is higher than 150°C and lower than 195°C.

**6.** The method according to any one of Claims 1 to 5, wherein the polyfluorocarboxylic acid has a fluorinated alkyl group of 4 to 16 carbon atoms.

**7.** The method according to any one of Claims 1 to 6, wherein the polyfluorocarboxylic acid is a perfluorocarboxylic acid or a salt thereof.

**8.** The method according to Claim 7, wherein the perfluorocarboxylic acid is perfluorooctanoic acid.

**9.** The method according to any one of Claims 1 to 8,

wherein a decomposition product of the polyfluorocarboxylic acid is a fluorinated compound represented by the following formula (D1),

$$Rf\text{-}H \qquad (D1)$$

wherein Rf is a fluorinated alkyl group of 3 to 15 carbon atoms which optionally have an ethereal oxygen atom.

## INTERNATIONAL SEARCH REPORT

| International application No. |
| --- |
| **PCT/JP2022/019051** |

### A. CLASSIFICATION OF SUBJECT MATTER

**C07C 17/363**(2006.01)i; **A62D 3/40**(2007.01)i; **C07C 19/08**(2006.01)i
FI:   C07C17/363; C07C19/08; A62D3/40

According to International Patent Classification (IPC) or to both national classification and IPC

### B. FIELDS SEARCHED

Minimum documentation searched (classification system followed by classification symbols)

C07C17/00; A62D3/00; C07C19/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
| --- | --- | --- |
| X | JP 2003-267900 A (NATIONAL INSTITUTE OF ADVANCED INDUSTRIAL & TECHNOLOGY) 25 September 2003 (2003-09-25) claims 1-11, paragraph [0015], examples 1-4 | 1-9 |
| A | JP 2003-040805 A (NATIONAL INSTITUTE OF ADVANCED INDUSTRIAL & TECHNOLOGY) 13 February 2003 (2003-02-13) claims 1-5 | 1-9 |
| A | JP 2006-169146 A (DAIKIN INDUSTRIES, LTD.) 29 June 2006 (2006-06-29) claims 1-11 | 1-9 |
| A | JP 2008-285449 A (NATIONAL INSTITUTE OF ADVANCED INDUSTRIAL & TECHNOLOGY) 27 November 2008 (2008-11-27) claims 1-4 | 1-9 |
| A | JP 2006-306736 A (NATIONAL INSTITUTE OF ADVANCED INDUSTRIAL & TECHNOLOGY) 09 November 2006 (2006-11-09) claims 1-6 | 1-9 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **12 July 2022** | **26 July 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2022/019051**

| Patent document cited in search report | | | Publication date (day/month/year) | Patent family member(s) | Publication date (day/month/year) |
|---|---|---|---|---|---|
| JP | 2003-267900 | A | 25 September 2003 | (Family: none) | |
| JP | 2003-040805 | A | 13 February 2003 | (Family: none) | |
| JP | 2006-169146 | A | 29 June 2006 | (Family: none) | |
| JP | 2008-285449 | A | 27 November 2008 | (Family: none) | |
| JP | 2006-306736 | A | 09 November 2006 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021077490 A
- JP 2006306736 A
- JP 2006169146 A
- JP 2008285449 A

**Non-patent literature cited in the description**

- *Journal of Fluorine Chemistry,* 2005, vol. 126, 1510-1516